# EUROPEAN PATENT SPECIFICATION

(11) **EP 3 634 943 B1**
(45) Date of publication and mention of the grant of the patent: **09.09.2020**
(21) Application number: 18779608.1
(22) Date of filing: 24.09.2018
(51) Int. Cl.: C07C 229/26, C07C 249/02, C07C 251/24, C07C 227/18

(54) **METHOD FOR PREPARATION OF DIFLUOROMETHYLORNITHINE**
VERFAHREN ZUR HERSTELLUNG VON DIFLUORMETHYLORNITHIN
PROCÉDÉ DE PRÉPARATION DE DIFLUOROMÉTHYLORNITHINE

(30) Priority: 25.09.2017 EP 17192830; 25.09.2017 US 201762562546 P; 15.01.2018 EP 18151701; 18.09.2018 EP 18194996
(43) Date of publication of application: 15.04.2020
(73) Proprietor: Lonza Ltd, 3930 Visp (CH)
(72) Inventor: GUTMANN, Bernhard, 3930 Visp (CH); BERSIER, Michael, 3938 Ausserberg (CH); HANSELMANN, Paul, 3902 Brig-Glis (CH); KAPPE, Christian Oliver, 8010 Graz (AT); KOECKINGER, Manuel, 8010 Graz (AT); HONE, Christopher, 8010 Graz (AT)
(86) International application number: PCT/EP2018/075724
(87) International publication number: WO 2019/057951

(56) References cited:
- US-A- 4 330 559
- US-A- 4 413 141
- US-B1- 6 998 502
- BEY, P. ET AL.: "Direct Synthesis of alpha-Halogenomethyl-alpha-amino Acids from the Parent alpha-Amino A", JOURNAL OF ORGANIC CHEMISTRY, vol. 44, 1979, pages 2732-2742, XP002775665,
- NIELSEN, O.J.: "HFC-23 tilbage som vigtig drivhusgas", DANSK KEMI, vol. 94, no. 8, 2013, pages 12-14, XP009501602,
- KALYANAM, N. ET AL.: "A novel reaction cascade leading to a spontaneous intramolecular dipolar cycloaddition through simultaneous generation of 1,3-dipole and dipolarophile", TETRAHEDRON LETTERS, vol. 54, 2013, pages 5155-5158, XP002786022,
- KÖCKINGER, M. ET AL.: "Utilization of fluoroform for difluoromethylation in continuous flow: a concise synthesis of alpha-difluormethyl-amino acids", GREEN CHEMISTRY, vol. 20, 30 October 2017 (2017-10-30), pages 108-112, XP002786023,

## Description

The invention discloses a method for preparation of protected difluoromethylornithine by a difluoromethylation of dibenzaldimine ornithine ester using CHF₃ and LiHMDS (lithium bis(trimethylsilyl)amide), which can be converted to a salt of unprotected difluoromethylornithine by a subsequent deprotection under acidic conditions in the presence of water.

### BACKGROUND OF THE INVENTION

2-Difluoromethyl-2,5-diaminopentanoic acid, also called Difluoromethylornithine (DFMO) or eflornithine, is an inhibitor of ornithine decarboxylase, an enzyme which is involved in polyamine formation in organisms.
Eflornithine is a medication used to treat African trypanosomiasis, which is sleeping sickness.

US 4,330,559 discloses in example 5 the preparation of DFMO in form of its hydrochloride monohydrate by deprotonation of the ornithine dibenzaldimine methyl ester with butyl lithium and diisopropylamine providing in situ lithium diisopropylamide, which is followed by reaction with chlorodifluoromethane (HCFC-22 or R-22); DFMO in form of its hydrochloride monohydrate is provided after deprotection and hydrolysis with aqueous HCl. Reaction times reported are 3 h for the reaction with chlorodifluoromethane and 16 h for the deprotection by 12 N HCl. Yield was 37%.

US 4,413,141 discloses in example 3 the preparation of DFMO in form of its hydrochloride monohydrate by deprotonation of the dibenzylidene ornithine methyl ester with butyl lithium and diisopropylamine providing in situ lithium diisopropylamide, which is followed by reaction with chlorodifluoromethane (HCFC-22 or R-22); DFMO in form of its hydrochloride monohydrate is provided after deprotection and hydrolysis with aqueous HCl. Reaction times reported are 3 h for the reaction with chlorodifluoromethane and 16 h for the deprotection by 12 N HCl. Yield was 40%.

Philippe Bey et al., J. Org. Chem., 1979, 44, 2732-2742 discloses in example 9b the preparation of methyl 2-(Difluoromethyl)-2,5-bis(benzylideneamino) pentanoate by deprotonation of the ornithine dibenzaldimine methyl ester with lithium diisopropylamide, which is followed by reaction with chlorodifluoromethane (HCFC-22 or R-22).

R-22 is known for its ozone depletion potential which is no longer considered acceptable, for this reason R-22 is regulated by the Montreal Protocol and is being phased out in the EU and US.

There was a need to find a method for preparation of DFMO, which does not need a substance which falls under the Montreal Protocol, and which has high yields.

This need is met by a method which uses CHF₃, which is not regulated by the Montreal Protocol. The method shows a higher yield of over 70%, even of over 80%, compared with the yields disclosed in US 4,330,559 or in US 4,413,141, the reaction times are significantly reduced. CHF₃ has different reactivity than R-22 and does not react under the reaction conditions of example 5 of US 4,330,559, of example 3 of US 4,413,141 or example 9b of Philippe Bey et al., J. Org. Chem., 1979, 44, 2732-2742. Therefore CHF₃ needed different reaction conditions than R-22 resulting in the method of instant invention being different from those disclosed methods.

The following abbreviations are used, if not otherwise stated:
- DFMO: difluoromethylornithine, eflornithine, MW 182 g/mol, compound of formula (3)
- HCFC-22: chlorodifluoromethane
- LiHMDS: lithium bis(trimethylsilyl)amide
- Me-THF: 2 methyltetrahydrofuran
- R-22: chlorodifluoromethane
- RT: room temperature
- THF: tetrahydrofuran

### SUMMARY OF THE INVENTION

Subject of the invention is a method for the preparation of compound of formula (II) 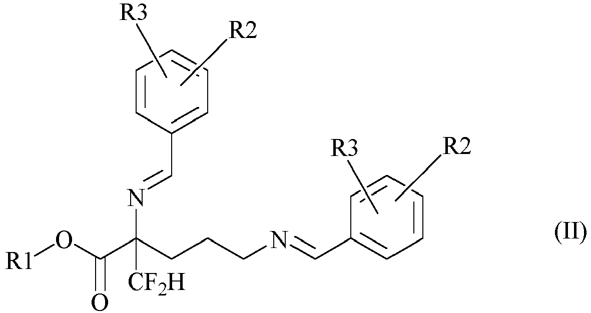 comprising a reaction REAC1, wherein compound of formula (I) is reacted with CHF₃ in the presence of LiHMDS (lithium bis(trimethylsilyl)amide); 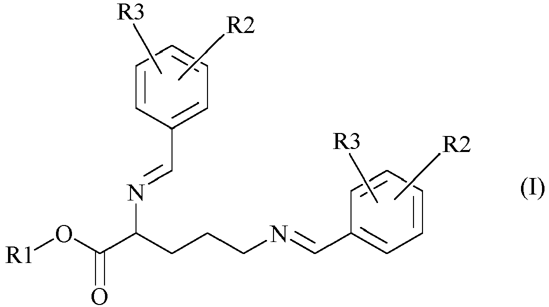
R1 is C₁₋₄ alkyl or benzyl;
R2 and R3 are identical or different and are selected from the group consisting of H, C₁₋₄ alkoxy, F, Cl, Br, I, NO₂, CN, CF₃, OH and C₁₋₄ alkyl.

### DETAILED DESCRIPTION OF THE INVENTION

Preferably, compound of formula (I) is compound of formula (I-CHIR), with R1, R2 and R3 as defined herein, also with all their embodiments.

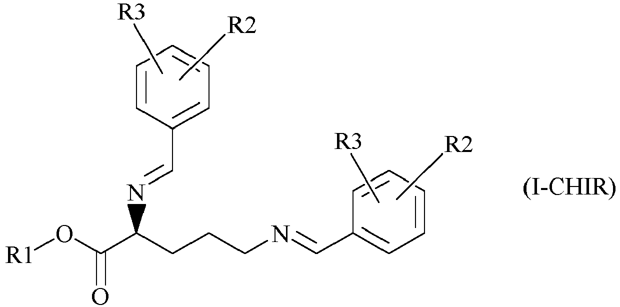

Preferably, R1 is methyl, ethyl, isopropyl, n-butyl or benzyl;
more preferably, R1 is methyl or ethyl;
even more preferably, R1 is methyl.

Preferably, R2 and R3 are selected from the group consisting of H, C₁₋₄ alkoxy, F, Cl, Br, CN, CF₃ and C₁₋₄ alkyl;
more preferably, R2 and R3 are selected from the group consisting of H, methoxy, ethoxy, Cl, Br, CN, CF₃, methyl and ethyl;
even more preferably, R2 and R3 are selected from the group consisting of H, methoxy, Cl, Br, CN, CF₃ and methyl;
especially, R2 and R3 are H, Cl, Br, or CF₃;
more especially, R2 and R3 are H or Cl.

Preferably, R2 and R3 are either identical or at least R3 is H.

Preferably, R2 is in para position;
more preferably, R2 is in para position and R3 is H.
In one embodiment, R2 and R3 are H.
In another embodiment, at least one of R2 or R3 is not H.

Particular embodiments of compound of formula (II) are selected from the group consisting of compound of formula (II-H), compound of formula (II-3-OMe), compound of formula (II-4-OMe), compound of formula (II-3-Cl), compound of formula (II-4-Cl), compound of formula (II-2-3-Cl), compound of formula (II-4-CN), compound of formula (II-4-Br), compound of formula (II-4-CF₃) and compound of formula (II-4-Me);
more in particular compound of formula (II-H), compound of formula (II-4-Cl), compound of formula (II-4-Br) and compound of formula (II-4-CF₃);
even more in particular compound of formula (II-H) and compound of formula (II-4-Cl);
with R1 as defined herein, also with all its embodiments, preferably with R1 being methyl.

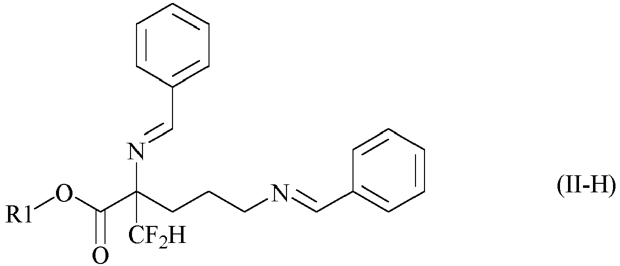

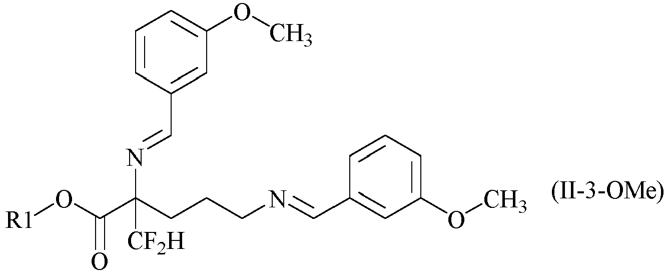

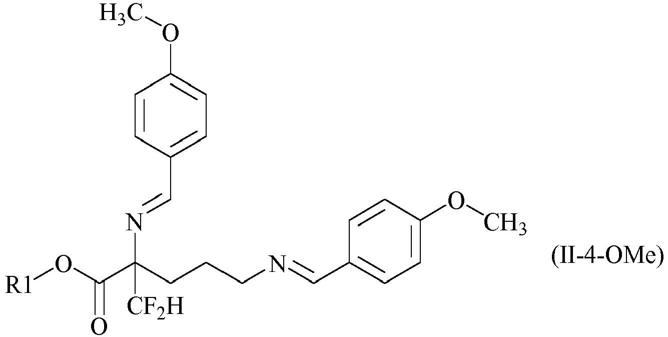

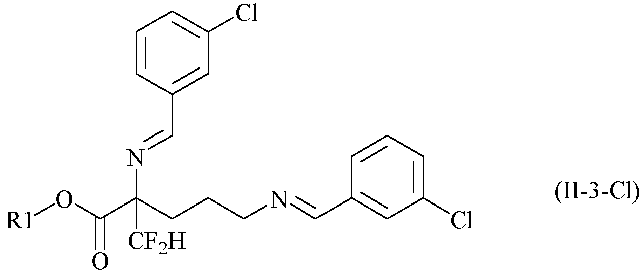

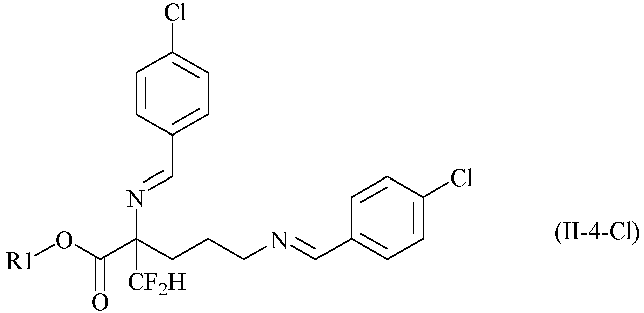

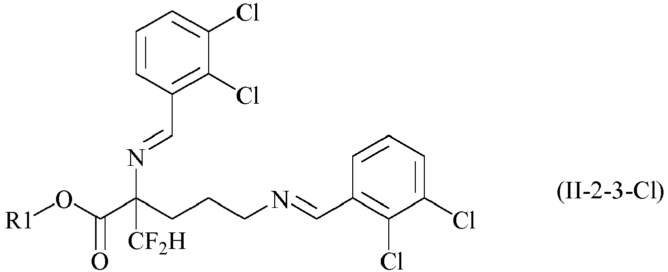

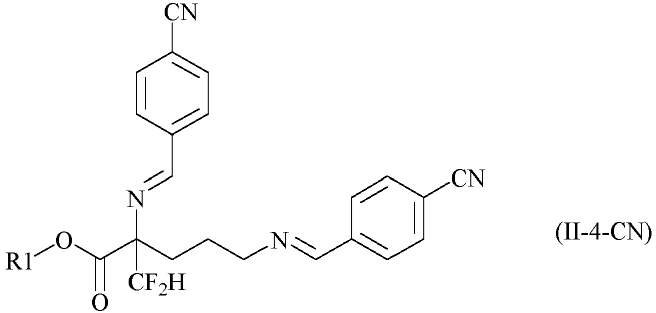

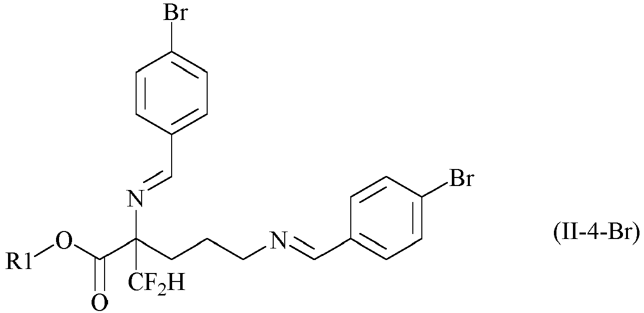

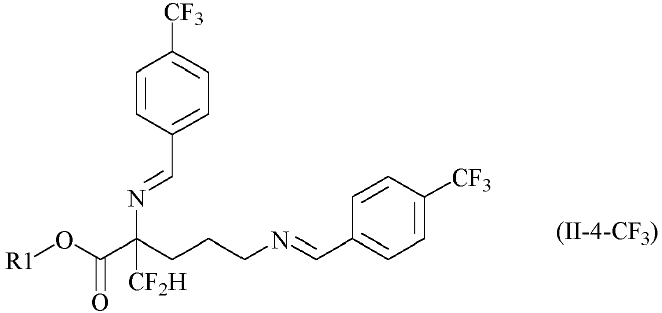

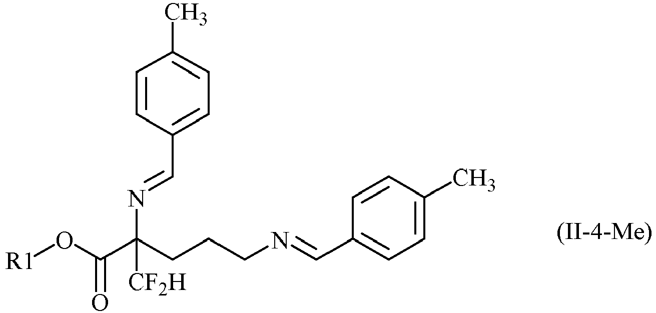

Particular embodiments of compound of formula (I) are selected from the group consisting of compound of formula (I-H), compound of formula (I-3-OMe), compound of formula (I-4-OMe), compound of formula (I-3-Cl), compound of formula (I-4-Cl), compound of formula (I-2-3-Cl), compound of formula (I-4-CN), compound of formula (I-4-Br), compound of formula (I-4-CF₃) and compound of formula (I-4-Me);
more in particular compound of formula (I-H), compound of formula (I-4-Cl), compound of formula (I-4-Br) and compound of formula (I-4-CF₃);
even more in particular compound of formula (I-H) and compound of formula (I-4-Cl);
with R1 as defined herein, also with all its embodiments, preferably with R1 being methyl.

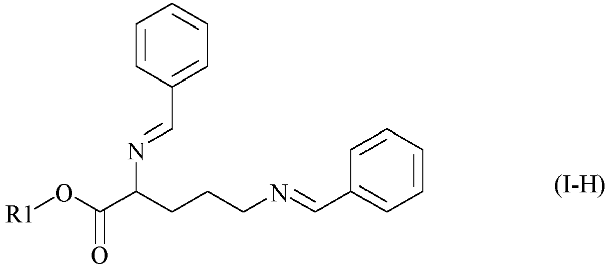

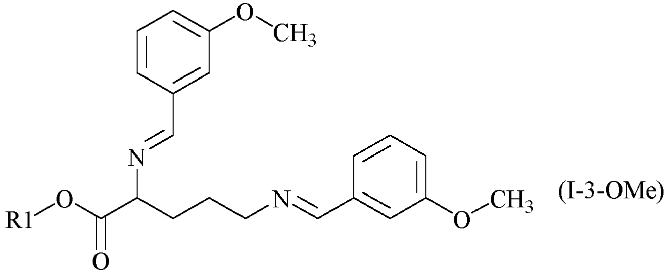

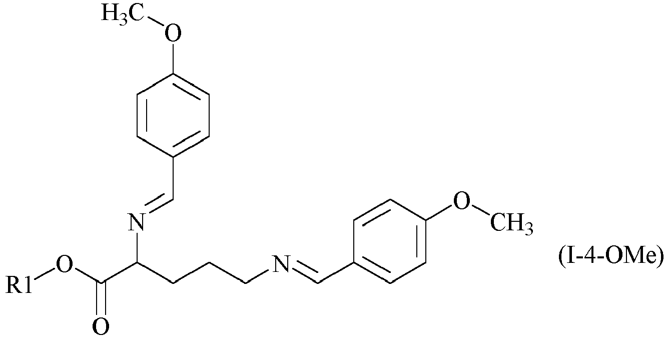

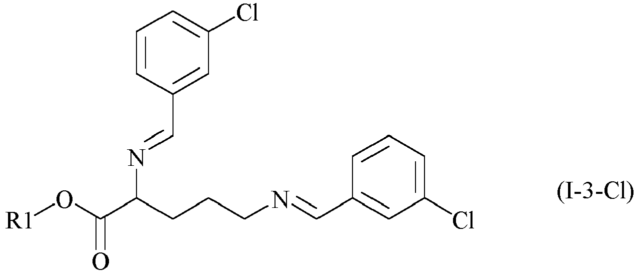

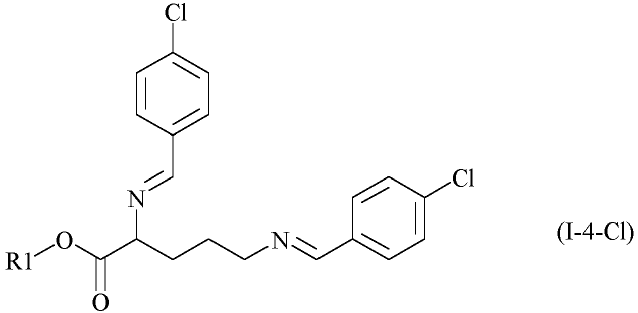

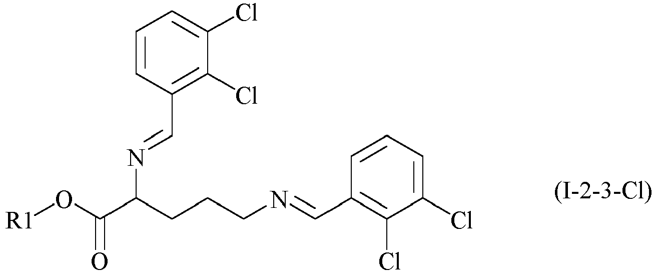

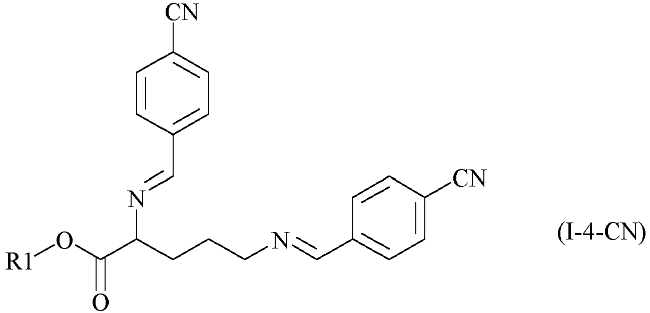

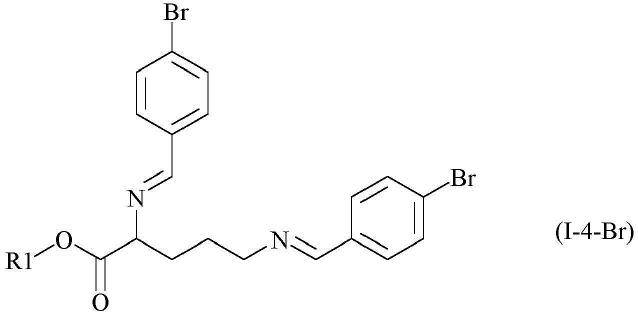

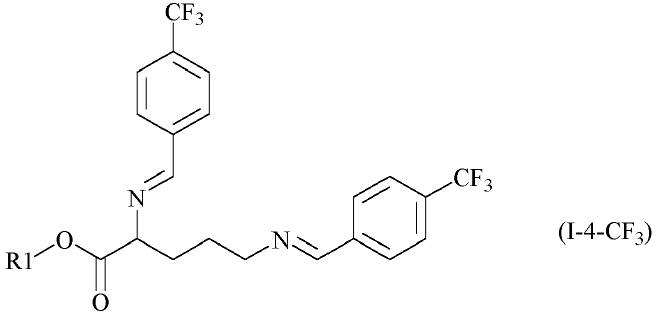

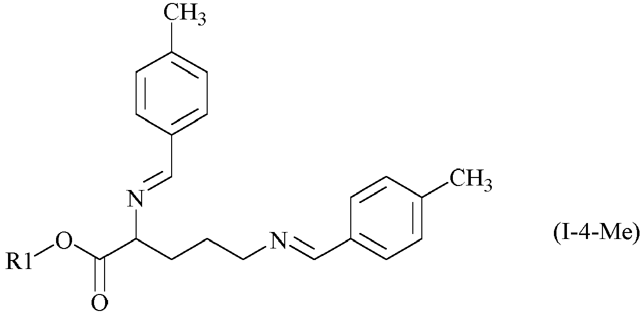

Specific embodiments of compound of formula (II) are compound of formula (2-H), compound of formula (2-4-Cl), compound of formula (2-4-Br) and compound of formula (2-4-CF₃);
preferably, compound of formula (2-H) and compound of formula (2-4-Cl).

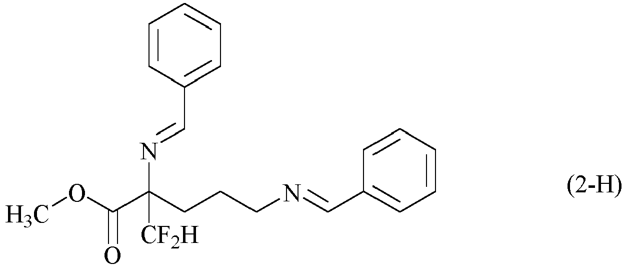

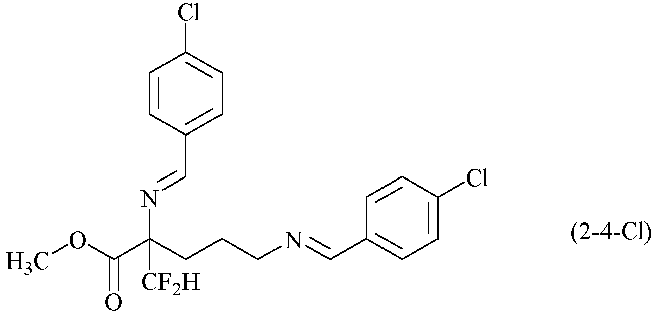

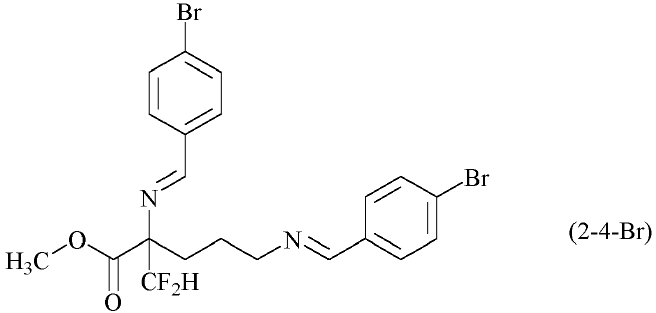

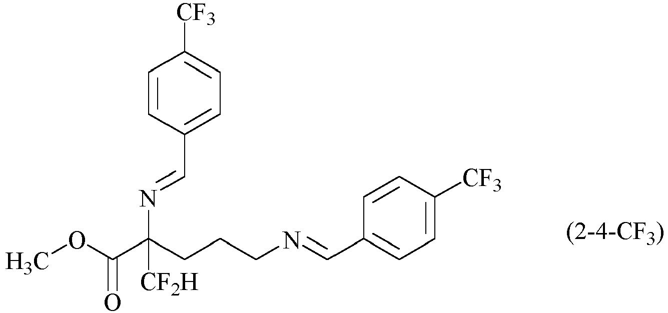

Specific embodiments of compound of formula (I) are compound of formula (1-H), compound of formula (1-4-Cl), compound of formula (1-4-Br) and compound of formula (1-4-CF₃);
preferably, compound of formula (1-H) and compound of formula (1-4-Cl).

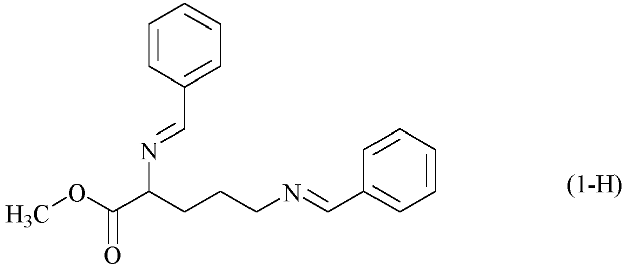

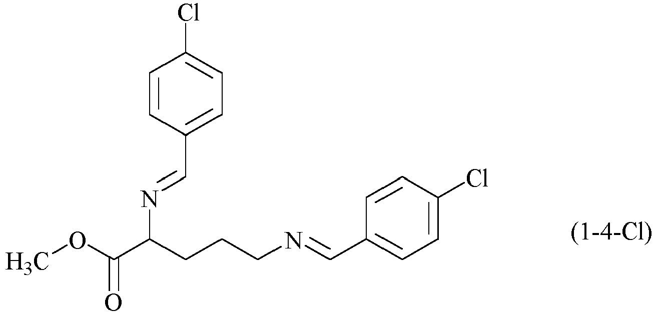

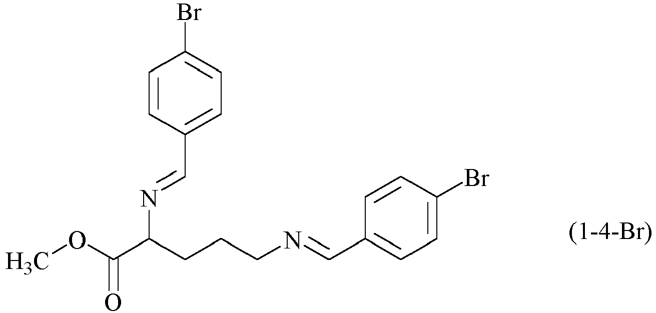

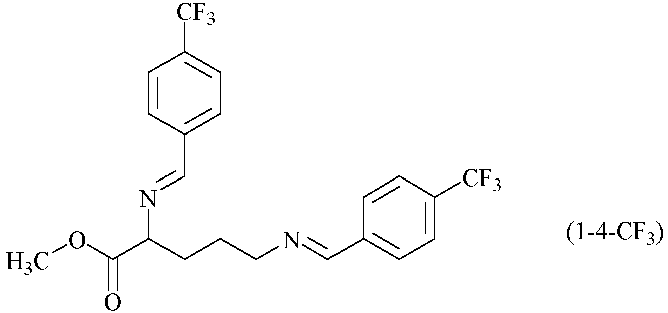

Preferably, in any of the compounds of formula (I) the alpha C atom next to the carboxy residue has the stereochemistry in analogy to the alpha C atom next to the carboxy residue of compound of formula (I-CHIR).

Preferably, the molar amount of CHF₃ in REAC1 is from 1 to 20 times, more preferably from 1.5 to 15 times, even more preferably from 2 to 15 times, of the molar amount of compound of formula (I).

In another embodiment, the molar amount of CHF₃ in REAC1 is from 1 to 20 times, more preferably from 1 to 15 times, even more preferably from 1 to 10 times, especially from 1 to 5 times, more especially from 1 to 3.5 times, even more especially from 1 to 2 times, in particular from 1 to 1.5 times, more in particular from 1 to 1.2 times, even more in particular from 1 to 1.1 times, of the molar amount of compound of formula (I).

Preferably, the molar amount of LiHMDS in REAC1 is from 1 to 20 times, more preferably from 1.5 to 15 times, even more preferably from 1.75 to 10 times, especially from 1.75 to 5 times of the molar amount of compound of formula (I).

REAC1 can be done in a solvent SOLV1, SOLV1 is a solvent that is compatible with LiHMDS (lithium bis(trimethylsilyl)amide), compatible means, that is does not react with LiMHDS, that it is inert towards LiHMDS;
preferably, SOLV1 is selected from the group consisting of THF, dioxane, methyl-THF and toluene; more preferably SOLV1 is THF or methyl-THF, even more preferably, SOLV1 is methyl-THF.

Preferably, methyl-THF is 2-methyl-THF.

Preferably, the weight of SOLV1 in REAC1 is from 1 to 100 times, more preferably from 2 to 50 times, even more preferably from 3 to 30 times, especially from 4 to 20 times, more especially from 4 to 15 times, of the weight of compound of formula (I).

Preferably, the reaction temperature of REAC1 is from -70 to 100 °C, more preferably from -50 to 80 °C, even more preferably from -30 to 60 °C, especially from -20 to 50 °C, more especially from -20 to 40 °C.

Preferably, the reaction time of REAC1 is from 1 min to 1 h, more preferably from 2 min to 45 min, even more preferably from 3 min to 30 min.

The pressure, under which REAC1 is done, can simply be the vapor pressure of the reaction mixture at the chosen reaction temperature at which REAC1 is done. REAC1 can also be done under elevated pressure, which can be applied by use of an inert gas such as nitrogen or argon, or by charging CHF₃ with a respective pressure. In case of a continuous reaction set up elevated pressure can also be realized by feeding the reactants against a back pressure regulation device, or elevated pressure can be realized by a combination of these measures.

Suitable back pressure regulation devices are known to the skilled person and are available on the market, such as from companies like Swagelok Company, Solon, Ohio, US, or Zaiput Flow Technologies, Scottsdale, Arizona, US. It should be a back pressure regulation device with a small dead volume, precise pressure control and large enough channel width to ensure a smooth flow without clogging.

Preferably, REAC1 is done under atmospheric pressure or under elevated pressure, such as from atmospheric pressure to 500 bar; more preferably, REAC1 is done at a pressure of from 2 bar to 500 bar, even more preferably of from 3 bar to 250 bar, especially of from 5 bar to 100 bar.

Preferably, REAC1 is done continuously. Compound of formula (I), LiHMDS and CHF₃ can be fed by pumping respective feeds through a respective continuously working mixing device MIXDEV, preferably this pumping is done against a back pressure regulation device, which holds the pressure in MIXDEV at a constant level.

Preferably, compound of formula (I) is mixed with LiHMDS, for this mixing also a continuously working mixing device can be use, then this mixture is mixed, preferably in in MIXDEV, with CHF₃, then REAC1 takes place.

MIXDEV can be any suitable device or installation which can be used for mixing fluids or for mixing fluids with gases for the case that under the reaction conditions CHF₃ is in a gaseous state; such suitable devices or installations are known in the state of the art, such as a common branch connection, e.g. a T or Y piece, a static mixing device or a micro reactor, preferably MIXDEV is a static mixing device or a micro reactor.

Static mixing devices, e.g. static mixers, are well established and widespread in all fields of chemical process technology. It is characteristically for static mixing devices, that, in contrast to dynamic mixing devices, only the media to be mixed are in motion. The liquids or gases are mixed by pump energy only, while the geometrically strong defined mixing elements in the static mixing devices remain in position. Companies such as Fluitec, Seuzachstrasse, 8413 Neftenbach, Switzerland, or Sulzer Ltd, Neuwiesenstrasse 15, 8401 Winterthur, Switzerland, are well known suppliers among others of such static mixing devices.

Micro reactors, also called micro structured reactors, are devices in which chemical reactions take place in a confinement with typical lateral dimensions below 1 mm; the most typical form of such confinement are micro channels. A micro reactor is a continuous flow reactor. They have been successfully applied in lab, pilot and production scale. E.g. the Fraunhofer Institute for Chemical Technology ICT, Joseph-von-Fraunhofer Strasse 7, 76327 Pfinztal, Germany, develops and offers such micro reactors.

Preferably, the static mixing device has the form of a tube or a plate containing means that present obstacles for the flow of the reaction mixture and thereby effecting the mixing of the components.

Preferably, the micro reactor contains micro channels which are arranged in such a way as to effect the mixing of the components.

Depending on the geometry of MIXDEV, of the velocity of the flow of the components and of the resulting residence time during which the reaction mixture is in MIXDEV, the reaction mixture exiting from MIXDEV can pass through a further device that is providing additional residence time after the mixing itself, such as a tube, thereby additional residence time, that is additional reaction time, in which REAC1 can take place, can be realized.

After REAC1, compound of formula (II) can be isolated by standard methods such as evaporation of volatile components, distillation, preferably under reduced pressure, extraction, washing, drying, concentration, crystallization, chromatography and any combination thereof, which are known per se to the person skilled in the art.

Preferably, compound of formula (I) is prepared by a reaction REAC0, wherein compound of formula (V) is reacted with compound of formula (IV); with R1, R2 and R3 as defined herein, also with all their embodiments.

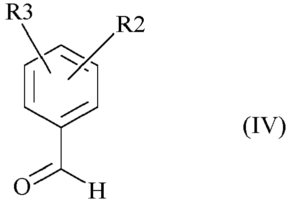

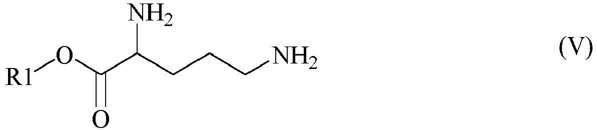

Preferably, compound of formula (V) is the respective ester of ornithine.

Preferably, compound of formula (V) is used in form of its dihydrochloride.

Preferably, the molar amount of compound of formula (IV) in REAC0 is from 2 to 10 times, more preferably from 2 to 5 times, even more preferably from 2 to 4 times, especially from 2 to 3 times, of the molar amount of compound of formula (V).

Preferably, REAC0 is done in the presence of a base BASE0; BASE0 is preferably a (C₁₋₄ alkyl)₃ amine, more preferably, BASE0 is triethylamine.

Preferably, the molar amount of BASE0 in REAC0 is from 2 to 5 times, more preferably from 2 to 4 times, even more preferably from 2 to 3 times, of the molar amount of compound of formula (V).

More preferably, compound of formula (V) is used in form of its dihydrochloride and REAC0 is done in the presence of BASE0.

REAC0 can be done in a solvent SOLV0;
preferably, SOLV0 is chloroform or dichloromethane, more preferably, SOLV0 is dichloromethane.

Preferably, the weight of SOLV0 in REAC0 is from 4 to 100 times, more preferably from 5 to 50 times, even more preferably from 5 to 40 times, of the weight of compound of formula (V).

Preferably, the reaction temperature of REAC0 is from -10 to 50 °C, more preferably from -5 to 40 °C, even more preferably from -5 to 30 °C.

Preferably, the reaction time TIME0 of REAC0 is from 1 to 48 h, more preferably from 5 to 24 h, even more preferably from 10 to 24 h.

Preferably, REAC0 is done under atmospheric pressure.

Preferably, REAC0 is done under inert atmosphere, such as nitrogen or argon atmosphere.

Preferably, when REAC0 is done in the presence of BASE0, BASE0 is mixed with a mixture of compound of formula (IV) and compound of formula (V).

More preferably, said mixing of BASE0 with the mixture of compound of formula (IV) and compound of formula (V) is done in a mixing time MIXTIME0, MIXTIME0 is from 1 min to 5 h, more preferably from 5 min to 2.5 h, even more preferably from 5 min to 2 h.

In another embodiment, MIXTIME0 is from 45 min to 5 h, more preferably from 45 min to 2.5 h, even more preferably from 45 min to 2 h, especially from 45 min to 1.5 h.

Preferably, MIXTIME0 is a part of TIME0; more preferably, said mixing is done at the beginning of TIME0.

Preferably, the reaction temperature during said mixing, that is during MIXTIME0, is from - 10 to 5 °C, more preferably from -5 to 5 °C, even more preferably from -5 to 2 °C, especially from -2 to 1 °C; and more preferably, after MIXTIME0, that is during the remaining TIME0, the reaction temperature is raised to room temperature.

REAC0 according to the invention provides for a low content of compound of formula (LACT), with R2 and R3 as defined herein, also with all their preferred embodiments.

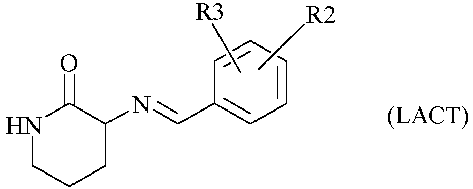

Preferably, compound of formula (LACT) is substituted as desired for REAC1; embodiments of compound of formula (LACT) are compound of formula (LACT-4-H) and compound of formula (LACT-4-Cl).

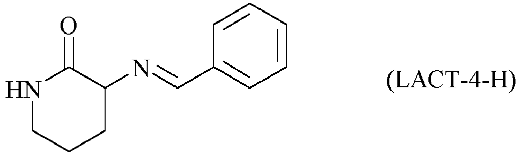

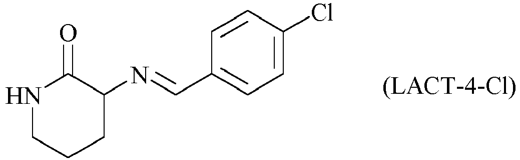

After REAC0 compound of formula (I) can be isolated by standard methods known to the skilled person, such a evaporation of solvent, removal of any salt of BASE0 that has formed during REAC0, preferably said removal by precipitation and filtration, concentration of the product in vacuum, crystallization.

Compound of formula (II) can be converted to compound of formula (3) by a deprotection of compound of formula (II);
preferably by a deprotection of compound formula (II) with an acid in the presence of water. Therefore, further subject of the invention is a method for the preparation of compound of formula (3) 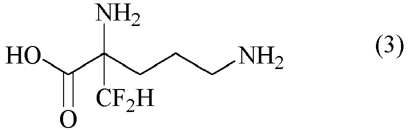 comprising REAC1 and a deprotection of compound of formula (II);
with REAC1 and compound of formula (II) as defined herein, also with all their embodiments.

Preferably, the deprotection is done with an acid and in the presence of water;
preferably, the acid used for the deprotection is ACID2, ACID2 is HCl, H₂SO₄ or acetic acid; more preferably, ACID2 is HCl.

Compound of formula (3) may be obtained as a salt with one or two equivalents of ACID2, such as compound of formula (III-1ACID2), compound formula (III-2ACID2), in particular compound of formula (3-1HCl), compound formula (3-2HCl).

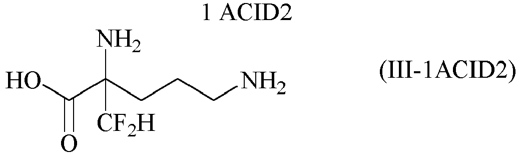

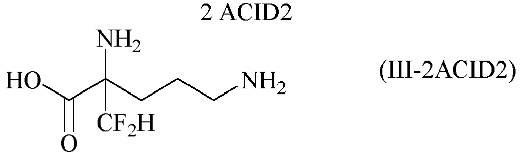

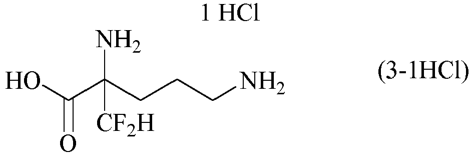

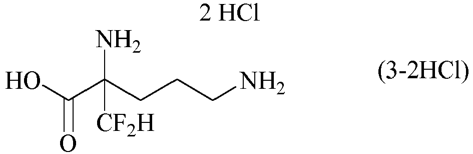

Compound of formula (3), compound of formula (III-1ACID2) and compound of formula (III-2ACID2) may also crystalize as a hydrate, such as for example as a monohydrate or a dihydrate, for example as compound of formula (3-1HCl-1H2O), the monohydrochloride monohydrate.

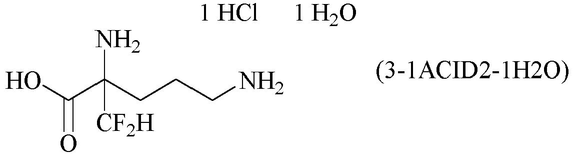

Preferably, Compound of formula (II), that is deprotected, has been prepared by REAC1.

Compound of formula (II) is protected by three protecting residues, that are the ester residue and the two benzylidene residues. The removal of the three protecting residues by the deprotection can be done consecutively in two or three consecutive deprotection reactions, an example for a consecutive removal of the protecting groups would be the situation when R1 is benzyl and R2 and R3 are benzylidene residues, then R1 can be removed catalytically whereas R2 and R3 can be removed by acidic hydrolysis, or the deprotection of all three protecting residues can be done in one deprotection reaction, for example hen R1 is a C1-4 alkyl residue and R2 and R3 are said benzylidene resisues, then all three protecting group can be removed simultaneously by an acidic hydrolysis. So the deprotection of compound of formula (II) comprises the removal of the alkoxy residue R1-O- in the ester residue to provide the respective acid residue and comprises the removal of the two benzylidene residues from the amino residues.

Preferably, deprotection is done in one reaction REAC2, that is all protecting residues are removed simultaneously in this one reaction REAC2.

Preferably, the molar amount of ACID2 for the deprotection is at least 2 times, more preferably at least 3 times, even more preferably at least 4 times, of the molar amount of compound of formula (II).

Preferably, the molar amount of water for the deprotection is at least 3 times, more preferably at least 4 times, even more preferably at least 5 times, of the molar amount of compound of formula (II).

Preferably, ACID2 is used in form of an aqueous ACID2;
preferably, the aqueous ACID2 that is used for the deprotection is at least 1 N, more preferably at least 2 N, even more preferably at least 3 N, aqueous ACID2;
preferably, the aqueous ACID2 that is used for the deprotection is from 1 to 13 N, more preferably from 2 to 13 N, even more preferably from 3 to 13 N, aqueous ACID2.

In case that ACID2 is HCl, then preferably HCl conc. is used, such as aqueous HCl 35 wt% or 12 N aqueous HCl.

Preferably, the reaction temperature of the deprotection is from 50 to 200 °C, more preferably from 75 to 180 °C, even more preferably from 95 to 160 °C.

In another embodiment, the reaction temperature of the deprotection is preferably from 50 to 200 °C, more preferably from 75 to 180 °C, even more preferably from 95 to 180 °C, especially from 110 to 180 °C, more especially from 130 to 180°C, even more especially from 140 to 180°C, in particular from 140 to 170°C; preferably these ranges of the reaction temperature are applied when the deprotection is done continuously.

Preferably, the reaction time of the deprotection is from 1 min to 48 h, more preferably from 10 min to 24 h, even more preferably from 30 min to 12 h, especially from 30 min to 6 h, more especially from 30 min to 2 h.

Preferably, the deprotection is done under atmospheric pressure or under elevated pressure, such as from atmospheric pressure to 500 bar; more preferably such as from atmospheric pressure to to 500 bar, even more preferably such as from atmospheric pressure to 250 bar, especially such as from atmospheric pressure to 100 bar.

Elevated pressure can be applied by use of an inert gas such as nitrogen or argon.

The deprotection, in particular REAC2, when the deprotection is done in one reaction REAC2, can be done continuously. REAC1 and the deprotection can both be done continuously and consecutively, this can be done by using the reaction mixture from REAC1 directly as feed for the deprotection.

The pressure, under which the deprotection is done, can be the vapor pressure of the reaction mixture at the chosen temperature, or, when the deprotection is done continuously, especially when REAC1 and the deprotection are done continuously and consecutively, then the pressure, under which the deprotection is done, can be the pressure chosen and set by the back pressure regulation device against which the pumping of the feeds is done, as described herein.

After the deprotection, compound of formula (3) can be isolated by standard methods such as evaporation of volatile components, extraction, washing, drying, concentration, crystallization, chromatography and any combination thereof, which are known per se to the person skilled in the art.

Further subject of the invention are compound of formula (2-4-Cl), compound of formula (2-4-Br) and compound of formula (2-4-CF₃).

### Examples

L-ornithine methyl ester dihydrochloride: purchased from Fluorochem Ltd, www.fluorochem.co.uk, Catalogue number 429762, (S)-Methyl 2,5-diaminopentanoate dihydrochloride, CAS 40216-82-8
4-chlorobenzaldehyde: 97% purity, Sigma Aldrich
lithium bis(trimethylsilyl)amide: 1 M solution in THF, Sigma Aldrich
fluoroform: 99.995% purity, Messer Group GmbH, Bad Soden, Germany
2 methyltetrahydrofuran anhydrous: ≥99% purity, inhibitor free, Acros Organics BVBA, Belgium
tetrahydrofuran anhydrous: ≥99%, Acros Organics BVBA, Belgium
concentrated HCl: 35% in water, VWR International Ltd., UK
dichloromethane: >98% purity, VWR International Ltd., UK
chloroform: 99.2% purity, VWR International Ltd., UK
trimethylamine: ≥99%, Riedel de Haën (Honeywell Specialty Chemicals Seelze GmbH, Germany)

### General

¹H and ¹³C NMR spectra were recorded on a 300 MHz instrument. Chemical shifts (delta) are expressed in ppm downfield from TMS as an internal standard. The letters s, d, t, q, and m are used to indicate singlet, doublet, triplet, quadruplet, and multiplet, respectively. Trifluorotoluene was used as internal standard for ¹⁹F NMR.

### Example 1

### Methyl (S)-2,5-bis((benzylidene)amino) pentanoate, compound of formula (1-H)

A dry 20 mL vessel with magnetic stirring bar was charged with 1.530 g (7.00 mmol, 1.0 eq) L-ornithine methyl ester dihydrochloride, sealed and flushed with argon three times. 7 mL CHCl₃ was added to afford a 1 M solution followed by the addition of 1.42 mL (13.93 mmol, 1.99 eq) benzaldehyde. The resulting stirred solution was cooled to 0 °C. After adding 2.13 mL (15.4 mmol, 2.2 eq) triethylamine over 10 min, the mixture was gradually warmed to room temperature and stirred for 24 h at RT. The reaction mixture was filtered through Na₂SO₄ and the obtained light yellow filtrate was concentrated under vacuum and treated with Et₂O to precipitate Et₃N·HCl. The formed colourless precipitate was filtered off and the obtained filtrate was concentrated in vacuum to give compound of formula (1). Yield: 2.2136 g (6.87 mmol, 98%), yellow viscous oil.
¹H-NMR (300 MHz, CDCl₃): delta = 7.68-7.56 (m, 4H), 7.47-7.29 (m, 12H), 7.20-7.08 (m, 4H), 4.10 (dd, ³J_{HH}= 8.0 Hz, ³J_{HH}= 5.2 Hz, 1H), 3.71 (s, 3H), 3.33 (t, ³J_{HH} = 6.8 Hz, 2H), 2.07-1.88 (m, 2H), 1.79-1.54 (m, 2H).

### Example 2

### alpha difluoromethylornithine dihydrochloride (DFMO), compound of formula (3-2HCl)

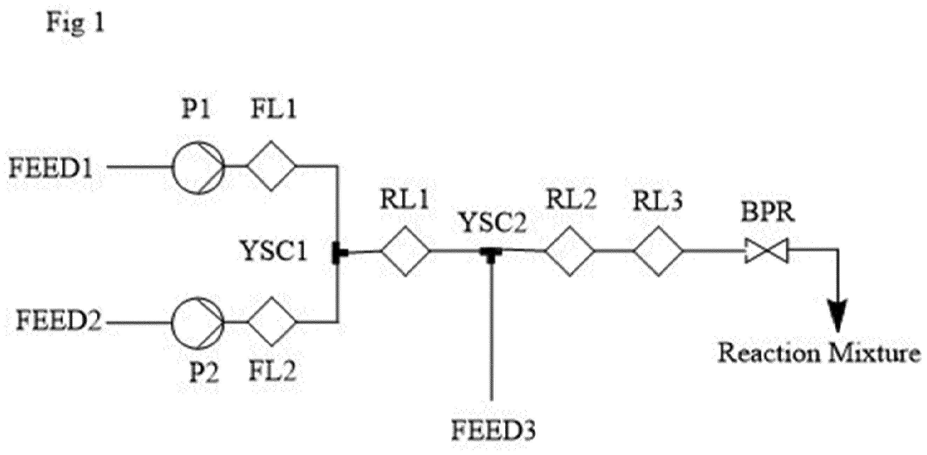

Fig. 1 shows an exemplary reactor set up that can be used to prepare continuously a compound of formula (II).

The complete reactor setup as shown in Fig 1 was flushed with pure solvent by pumping dry THF with the pump P1 and a flow rate FR1 of 250 microliter/min and with the pump P2 and a flow rate FR2 of 250 microliter/min through respective injection loops FL1 (0.8 mm inner diameter, 1.59 mm outer diameter, internal volume 2.0 mL) and FL2 (0.8 mm inner diameter, 1.59 mm outer diameter, internal volume 2.5 mL) via a first Y shaped connector YSC1 into a first residence loop RL1 (1/16 inch outer diameter; 0.8 mm inner diameter; 2.0 mL residence volume) and onwards through a second residence loop RL2 (1/16 inch outer diameter; 0.8 mm inner diameter; 6.1 mL residence volume), a third residence loop RL3 (1/8 inch outer diameter; 1.6 mm inner diameter; 2.0 mL residence volume) and a device for back pressure regulation BPR, between RL1 and RL2 additionally CHF₃ was fed from a pressurized bottle into the stream via a second Y shaped connector YSC2 with a flow rate FR3 of 8.3 mL/min, until the internal pressure of the set up reached 12 bar, this process took ca. 10 min.

Compound of formula (1), prepared according to example 1, (1.00 mmol), was dissolved in neat THF and diluted to 2.00 mL (0.5 mol/L, 1 eq) with THF and served as feed FEED1 via pump P1.

A LiHMDS solution (1.0 mol/L, 2.5 mL, 2 eq) in THF was used as feed FEED2 via pump P2.

Pumping of FEED1 was started 1 min prior to FEED2. Both feeds were pumped through their respective FL1 and FL2 through YSC1 which was located in a cooling bath with the temperature of -30 °C.

Flow rate FR1 and flow rate FR2 were 250 microliter/min.

The combined mixture then passed through RL1, which was located in a cooling bath having a temperature of -30°C. The resulting reaction mixture stream was brought to contact with a third feed FEED3, which was CHF₃, and which was fed at FR3 of 8.3 mL/min (gas flow at normal conditions; corresponds to 3 eq of CHF₃) into YSC2, which was located in a cooling bath having a temperature of -15°C, the resulting reaction mixture stream then passed on through RL2, which was located in a cooling bath having a temperature of -15°C, exiting TR2 the reaction mixture stream was then warmed by its passing through RL3, which was located in a bath having a temperature of 25°C, and was collected after having passed through BPR, that held the pressure in the set up at 12 bar. The yield as determined by ¹⁹F-NMR in this reaction mixture, which had been collected after having passed through BPR, was > 95%.

The dimensions of TR1, TR2 and TR3 together with the flow rates FR1, FR2 FR3 resulted in a residence time RES1 in TR1 of 4 min, in a residence time RES2 in TR2 of 12 min and in a residence time RES3 in TR3 of 4 min.

The reaction mixture comprising compound of formula (2) was dried in vacuum and diluted with 10 mL Et₂O. After a filtration the filtrate was concentrated in vacuum and treated with 10 mL 6 N HCl. The reaction mixture was heated to 150 °C for 45 min in the microwave reactor (Biotage Initiator+ single mode microwave instrument) and washed with Et₂O (2 times with 20 mL). The aqueous layer was mixed with activated carbon which was subsequently filtered off. After concentrating the crude product in vacuum it was recrystallized from MeOH/EtOH.

Yield of compound of formula (3-2HCl): 194 mg (0.76 mmol, 76%) colourless powder mp 228 °C
¹H-NMR (300.36 MHz, D₂O): delta = 6.33 (t, ²J_{HF} = 53.2 Hz, 1H), 3.02 (t, ³J_{HH} = 7.6 Hz, 2H), 2.16-1.53 (m, 4H)
¹³C NMR (75 MHz, D₂O): delta = 168.8(d, ³J_{CF} = 5.9 Hz), 114.9 (dd, ¹J_{CF} = 248.4 Hz, ¹J_{CF} = 246.0 Hz), 65.1 (dd, ²J_{CF} = 21.3 Hz, ²J_{CF} = 16.6 Hz), 38.7, 27.7 (d, ³J_{CF} = 5.0 Hz), 20.9 ¹⁹F NMR (282 MHz, D₂O): delta = 126.83 (dd, ²J_{FF} = 281.6 Hz, ²J_{HF} = 52.6 Hz), -132.39 (dd, ²J_{FF} = 281.6 Hz, ²J_{HF} = 53.8 Hz)

### Example 3 to 8

Example 2 was repeated with the differences as given in Table 1. The yield was determined by ¹⁹F-NMR in the reaction mixture which was collected after having passed through BPR. ^{(x)} In examples 3 to 7 the FEED1 was 0.25 M N-benzylidene-protected methyl ornithine in THF.

| | **Table 1** | | | | | | | |
|---|---|---|---|---|---|---|---|---|
| **Ex** | **FEED1** | **FEED2** | **FEED3** | **RES1/RES2/RES3** | **T** | **LiHMDS** | **CHF₃** | **Yield** |
| | **ml/min** | **ml/min** | **ml/min** | **min** | **°C** | **eq** | **eq** | **%** |
| **2** | 0.25 | 0.25 | 8.3 | 4/12/4 | -15 | 2 | 3 | >95 |
| **3^{(x)}** | 1.0 | 1.0 | 33 | 1/3/1 | -15 | 4 | 6 | >95 |
| **4^{(x)}** | 0.8 | 1.2 | 27 | 1/3/1 | -15 | 6 | 6 | >95 |
| **5^{(x)}** | 1.0 | 1.0 | 22 | 1/3/1 | -15 | 4 | 4 | >95 |
| **6^{(x)}** | 1.0 | 1.0 | 44 | 1/3/1 | -15 | 4 | 8 | >95 |
| **7^{(x)}** | 1.0 | 1.0 | 33 | 1/3/1 | 25 | 4 | 6 | >95 |
| **8** | 0.8 | 1.2 | 27 | 1/3/1 | -15 | 3 | 3 | 87 |

### Example 9

### Various bisimine protected ornithine methyl esters, compound of formula (I)

Various protecting groups for the NH₂-residues of ornithine methyl ester were applied using different benzaldehyde analogues. As the obtained bisimines are not stable towards chromatography, the yield and purity was determined through ¹H NMR using nitromethane as internal standard.

### General experimental procedure:

A dry 10 mL round bottom flask with magnetic stirring bar was charged with (S)-methyl 2,5-diaminopentanoate dihydrochloride (0.50 g, 2.282 mmol) and the corresponding benzaldehyde derivative compound of formula (IV) (2 equiv, 4.564 mmol), sealed and flushed with argon three times. Chloroform (5 mL) was added and the stirred, colorless reaction mixture was cooled to 0 °C. After addition of triethylamine (632 microliter, 4.564 mmol, 2 equiv) over 10 min the reaction mixture was allowed to warm to room temperature and stirred for 18 h. The solvent was removed under reduced pressure. The slightly yellow residue was treated with Et₂O to precipitate Et₃N·HCl. The formed colorless precipitate was filtered off and the obtained filtrated was concentrated in vacuo. The residue was checked for yield and purity with ¹H NMR. Result are shown on Table 2.

| **Table 2** | | | |
|---|---|---|---|
| **Entry** | **Compound of formula (I) with R1 = OMe** | **Melting point [°C]** | **Yield [%](a)** |
| 1 | R2 = 3-OMe / R3 = H formula (I-3-OMe) | (b) | 65 |
| 2 | 4-OMe / R3 = H formula (I-4-OMe) | (b) | 88 |
| 3 | 3-Cl / R3 = H formula (I-3-Cl) | (b) | 81 |
| 4 | 4-Cl / R3 = H formula (1-4-Cl) | 89-91 | 92 |
| 5 | R2 = 2-Cl / R3 = 3-Cl formula (I-2-3-Cl) | (b) | 87 |
| 6 | 4-CN / R3 = H formula (I-4-CN) | (b) | 82 |
| 7 | 4-Br / R3 = H formula (1-4-Br) | 74 to 77 | 92 |
| 8 | 4-CF₃ / R3 = H formula (1-4-CF₃) | (b) | 93 |
| 9 | 4-Me / R3 = H formula (I-4-Me) | 66 to 69 | 85 |

| | | | |
|---|---|---|---|
| (a): Analyzed by ¹H NMR via internal standard (CH₃NO₂), corrected for purity (b) liquid at RT | | | |

### Example 10

### 4-Chlorobenzaldehyde Protected Ornithine Methyl Ester, compound of formula (1-4-Cl)

A dry 2 L round bottom flask with magnetic stirring bar was charged with (S)-methyl 2,5-diaminopentanoate dihydrochloride (25.0 g, 114 mmol) and 4-chlorobenzaldehyde (24.2 g, 240 mmol), sealed and flushed with argon three times. Anhydrous dichloromethane (750 mL) was added and then the reaction mixture was stirred. The colorless reaction mixture was cooled to 0 °C. After addition of triethylamine (33.2 mL, 240 mmol) over 1 h, the reaction mixture was allowed to warm to room temperature and was stirred for 18 h. The solvent was removed under reduced pressure. The off-white residue was treated with Et₂O to precipitate Et₃N·HCl. The formed colorless precipitate was filtered off and the obtained filtrate was concentrated in vacuo. The obtained off-white residue was recrystallized from petrol ether/EtOAc to provide the desired compound of formula (1-4-Cl) as colorless crystalline solid (41.1 g, 105 mmol, 92% yield)
mp. 89-92 °C
¹H NMR (500 MHz, CDCl₃) delta 8.26 (dd, J = 11.6, 5.6 Hz, 2H), 7.75-7.70 (m, 2H), 7.67-7.63 (m, 2H), 7.43-7.35 (m, 4H), 4.06 (dd, J = 8.4, 5.3 Hz, 1H), 3.76 (s, 3H), 3.65 (t, J = 6.7 Hz, 2H), 2.16 - 2.07 (m, 1H), 2.06 - 1.94 (m, 1H), 1.81 - 1.66 (m, 2H) ppm
¹³C NMR (75 MHz, CDCl₃) delta 172.4, 162.2, 159.9, 137.3, 136.6, 134.7, 134.2, 129.8, 129.3, 129.0, 128.9, 73.3, 61.2, 52.3, 31.3, 27.4 ppm

To determine stability of compound of formula (1-4-Cl), eight NMR tubes were charged with 100 mg of compound of formula (1-4-Cl) and 10 mg CH3NO2 as internal standard, sealed with a cap and stored at -30 °C. The purity of samples was then determined by ¹H NMR over the course of 10 days. There was no significant degradation of compound of formula (1-4-Cl) detected.

Therefore compound of formula (1-4-Cl) could be stored for prolonged periods of time under argon at low temperatures (-30 °C) without degradation, and it was bench stable at room temperatures for at least 2 weeks.

Water content was determined by Karl Fischer titration on a Metrohm Coulometer "Aquaster Combi Coulomat fritless", a concentration of 1475 ppm H₂O was found.

### Example 11 - alpha difluoromethylornithine monohydrochloride monohydrate (DFMO), compound of formula (3-1HCl-1H2O)

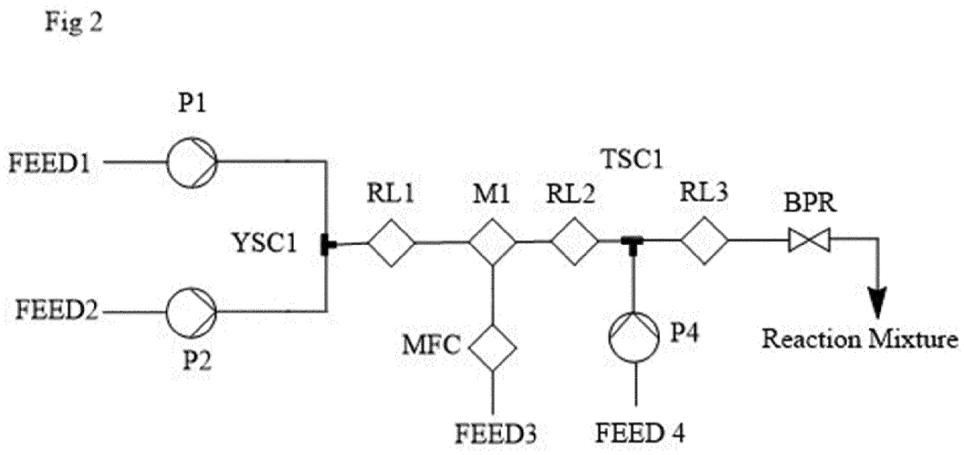

Fig. 2 shows an exemplary reactor set up that can be used to prepare continuously a compound of formula (3).

The reactor set up of Fig 2 consisted of three continuously working syringe pumps (2 × Asia Syrris, that are pump P1 and pump P2, 1 × HighTec Zang Syrdos, that is pump P4) to introduce a solution of compound of formula (1-4-Cl) in 2-MeTHF (FEED1), a commercial solution of LiHMDS (1.0 M in THF, Sigma-Aldrich) (FEED2) and concentrated HCl (35 wt% in water) (FEED4). FEED1, FEED2 and FEED4 were directly pumped through the syringe pumps. To start the experiment, the complete reactor setup was flushed by pumping dry 2-MeTHF with flow rates of FEED1 = 0.8 mL/min and FEED2 = 1.2 mL/min. HCl was introduced with FEED4 = 1 mL/min. Fluoroform was introduced into the reactor with a flow rate of 9.23 mL/min (1.05 equiv) using a calibrated mass flow controller (MFC) of Bronkhorst. The internal pressure of the reactor reached the target pressure of 12 bar after approximately 10 min.

Compound of formula (1-4-Cl) (100 mmol) was dissolved in neat 2-MeTHF and diluted to 200 mL in two volumetric flasks with 2-MeTHF (FEED1, 0.5 M). A LiHMDS solution (1.0 M, 800 mL) in THF was used as FEED2. FEED1 and FEED2 pumped directly through the syringe pumps were combined in a Y-shaped connector YSC1 (Y Assembly PEEK 1/4-28 0.040 in) in a cooling bath (-30 °C). The combined mixture went through a first residence loop RL1 at -30 °C (1/8 in outer diameter.; 0.8 mm inner diameter; residence volume V1 = 4.0 mL), before the mixture was combined with fluoroform in a Syrris Asia glass static mixer M1 (1 mL internal volume). The mixture then went through a second residence loop RL2 at 25 °C (1/8 in outer diameter; 0.8 mm inner diameter; residence volume V2 = 14 mL) and were mixed with FEED4 in a T-shaped connector TSC1 (T Assembly PTFE 1/4-28 0.040 in). The combined stream then went through a third residence loop RL3 at 160 °C (1/8 in. outer diameter; 0.8 mm inner diameter; residence volume V3 = 40 mL) and left the flow system through an adjustable back pressure regulator BPR (Zaiput BPR-10). The run was conducted for 4 h with a throughput of 24 mmol/h.

"The employed back pressure rose from 12 to 15 bar.

Residence time in the total reactor set up was 23 min.

The biphasic reaction mixture was collected for 3.5 h (84 mmol) and the aqueous phase was washed with Et₂O and concentrated under reduced pressure. The crude product was dissolved in a small amount of H₂O and the pH was adjusted to 4 with Et₃N. The resulting slurry was filtered and washed with cold EtOH abs. and CHCl₃. The residue was recrystallized from EtOH/H₂O to give alpha difluoromethylornithine monohydrochloride monohydrate as colourless powder. (17.05 g, 72.3 mmol, 86% yield).
Mp. 228 °C
¹H NMR (300.36 MHz, D₂O): delta = 6.46 (t, 2JHF = 52.8 Hz, 1H), 3.05 (t,3JHH = 7.6 Hz, 2H), 2.25-1.97 (m, 2H), 1.96-1.79 (m, 1H), 1.76-1.59 (m, 1H) ppm
¹³C NMR (75 MHz, D2O): delta = 167.8 (d, 3JCF = 6.4 Hz), 114.0 (dd, 1JCF = 249.7 Hz, 1JCF = 247.0 Hz), 64.5 (dd, 2JCF = 20.4 Hz, 2JCF = 18.7 Hz), 38.8 (d, 3JCF = 7.3 Hz), 31.6 (d, 4JCF = 3.2 Hz), 20.8 ppm
¹⁹F NMR (282 MHz, D2O): delta = -126.28 (dd, 2JFF = 283.5 Hz, 2JHF = 52.4 Hz), -131.76 (dd, 2JFF = 283.5 Hz, 2JHF = 52.4 Hz) ppm

Conversion from compound of formula (2-4-Cl) to its fully deprotected analogue was measured by ¹H NMR via integration of base line separated CHF₂-peaks of compound of formula (2-4-Cl) and its fully deprotected analogue of dried residue, conversion was > 99%.

## Claims

1. Method for the preparation of compound of formula (II) 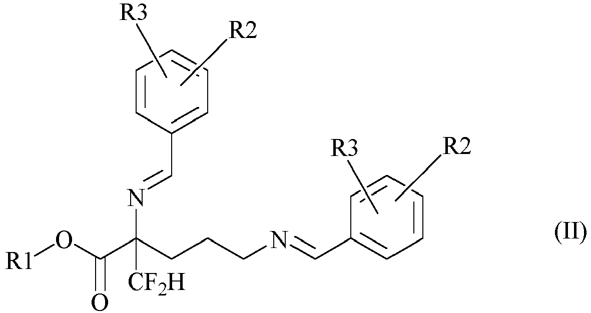 comprising a reaction REAC1, wherein compound of formula (I) is reacted with CHF₃ in the presence of lithium bis(trimethylsilyl)amide; 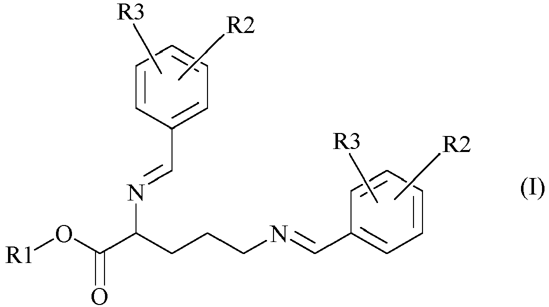
R1 is C₁₋₄ alkyl or benzyl;
R2 and R3 are identical or different and are selected from the group consisting of H, C₁₋₄ alkoxy, F, Cl, Br, I, NO₂, CN, CF₃, OH and C₁₋₄ alkyl.

2. Method according to claim 1, wherein
R1 is methyl, ethyl, isopropyl, n-butyl or benzyl.

3. Method according to claim 1 or 2, wherein
R2 and R3 are selected from the group consisting of H, C₁₋₄ alkoxy, F, Cl, Br, CN, CF₃ and C₁₋₄ alkyl.

4. Method according to one or more of claims 1 to 3, wherein
REAC1 is done in a solvent SOLV1, SOLV1 is a solvent that is compatible with lithium bis(trimethylsilyl)amide.

5. Method according to one or more of claims 1 to 4, wherein
REAC1 is done continuously.

6. Method according to one or more of claims 1 to 5, wherein
compound of formula (I) is prepared by a reaction REAC0, wherein
compound of formula (V) is reacted with compound of formula (IV);
with R1, R2 and R3 as defined in claim 1.

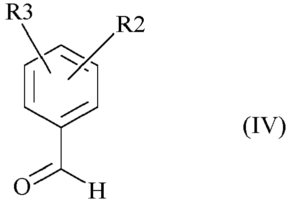

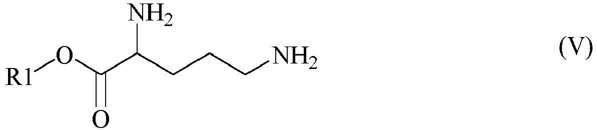

7. Method according to claim 6, wherein
REAC0 is done in the presence of a base BASE0.

8. Method according to claim 7, wherein
BASE0 is a (C₁₋₄ alkyl)₃ amine.

9. Method according to one or more of claims 6 to 8, wherein
REAC0 is done in a solvent SOLV0.

10. Method according to claim 9, wherein
SOLV0 is chloroform or dichloromethane.

11. Method for the preparation of compound of formula (3) 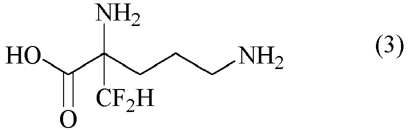 comprising REAC1 and a deprotection of compound of formula (II);
with REAC1 and compound of formula (II) as defined in claim 1.

12. Method according to claim 11, wherein
the deprotection is done with an acid and in the presence of water.

13. Method according to claim 12, wherein
the acid used for the deprotection is ACID2, ACID2 is HCl, H₂SO₄ or acetic acid.

14. Method according to one or more of claims 11 to 13, wherein
the deprotection is done continuously.

15. Method according to one or more of claims 11 to 14, wherein
REAC1 and the deprotection are both done continuously and consecutively.

16. Compound of formula (2-4-Cl), compound of formula (2-4-Br) and compound of formula (2-4-CF₃).

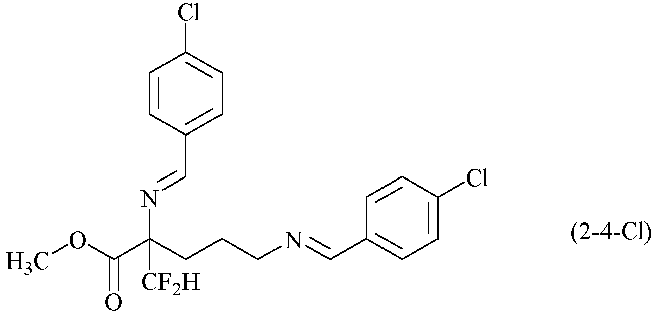

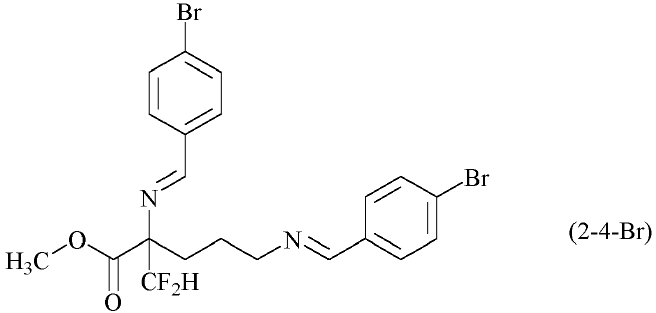

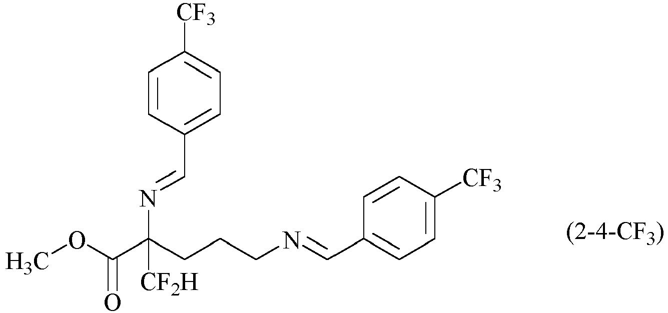

## Patentansprüche

1. Verfahren zur Herstellung einer Verbindung der Formel (II) 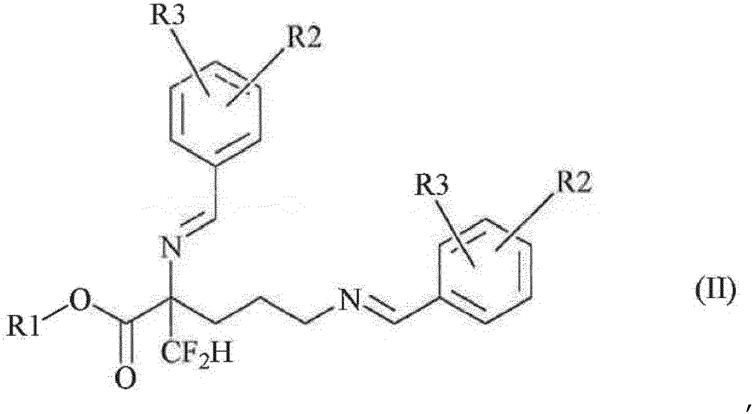 das eine Reaktion REAC1 umfasst, wobei die Verbindung der Formel (I) mit CHF₃ in Gegenwart von Lithium-bis(trimethylsilyl)amid umgesetzt wird; 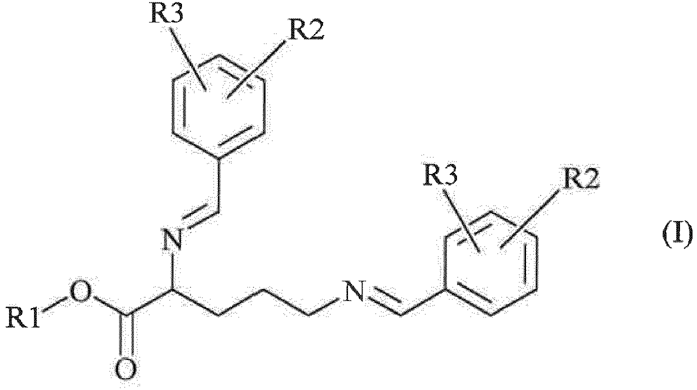
R1 für C₁₋₄-Alkyl oder Benzyl steht;
R2 und R3 gleich oder voneinander verschieden sind und aus der Gruppe bestehend aus H, C₁₋₄-Alkoxy, F, Cl, Br, I, NO₂, CN, CF₃, OH und C₁₋₄-Alkyl ausgewählt sind.

2. Verfahren nach Anspruch 1, wobei R1 für Methyl, Ethyl, Isopropyl, n-Butyl oder Benzyl steht.

3. Verfahren nach Anspruch 1 oder 2, wobei R2 und R3 aus der Gruppe bestehend aus H, C₁₋₄-Alkoxy, F, Cl, Br, CN, CF₃, und C₁₋₄-Alkyl ausgewählt sind.

4. Verfahren nach einem oder mehreren der Ansprüche 1 bis 3, wobei REAC1 in einem Lösungsmittel SOLV1 durchgeführt wird, wobei es sich bei SOLV1 um ein Lösungsmittel handelt, das mit Lithium-bis(trimethylsilyl)amid kompatibel ist.

5. Verfahren nach einem oder mehreren der Ansprüche 1 bis 4, wobei REAC1 kontinuierlich durchgeführt wird.

6. Verfahren nach einem oder mehreren der Ansprüche 1 bis 5, wobei die Verbindung der Formel (I) durch eine Reaktion REAC0 hergestellt wird, wobei eine Verbindung der Formel (V) mit einer Verbindung der Formel (IV) umgesetzt wird; wobei R1, R2 und R3 wie in Anspruch 1 definiert sind.

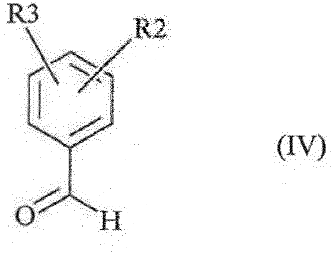

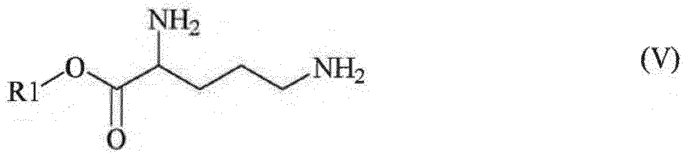

7. Verfahren nach Anspruch 6, wobei REAC0 in Gegenwart von einer Base BASE0 durchgeführt wird.

8. Verfahren nach Anspruch 7, wobei es sich bei BASE0 um ein (C₁₋₄-Alkyl)₃-amin handelt.

9. Verfahren nach einem oder mehreren der Ansprüche 6 bis 8, wobei REAC0 in einem Lösungsmittel SOLV0 durchgeführt wird.

10. Verfahren nach Anspruch 9, wobei es sich bei SOLV0 um Chloroform oder Dichlormethan handelt.

11. Verfahren zur Herstellung einer Verbindung der Formel (3) 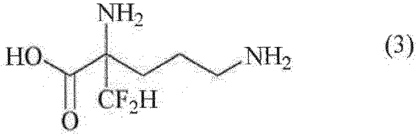 umfassend REAC1 und eine Entschützung der Verbindung der Formel (II);
wobei REAC1 und die Verbindung der Formel (II) wie in Anspruch 1 definiert sind.

12. Verfahren nach Anspruch 11, wobei die Entschützung mit einer Säure und in Gegenwart von Wasser durchgeführt wird.

13. Verfahren nach Anspruch 12, wobei es sich bei der für die Entschützung verwendeten Säure um ACID2 handelt, wobei es sich bei ACID2 um HCl, H₂SO₄ oder Essigsäure handelt.

14. Verfahren nach einem oder mehreren der Ansprüche 11 bis 13, wobei die Entschützung kontinuierlich durchgeführt wird.

15. Verfahren nach einem oder mehreren der Ansprüche 11 bis 14, wobei REAC1 und die Entschützung beide kontinuierlich und aufeinanderfolgend durchgeführt werden.

16. Verbindung der Formel (2-4-Cl), Verbindung der Formel (2-4-Br) und Verbindung der Formel (2-4-CF₃).

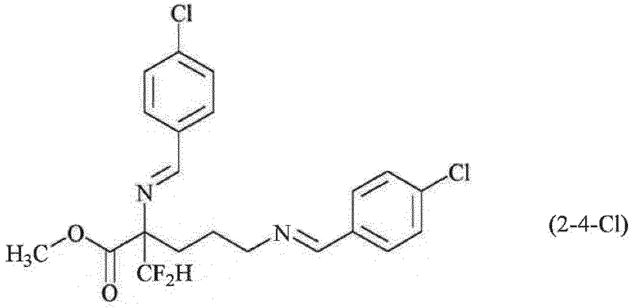

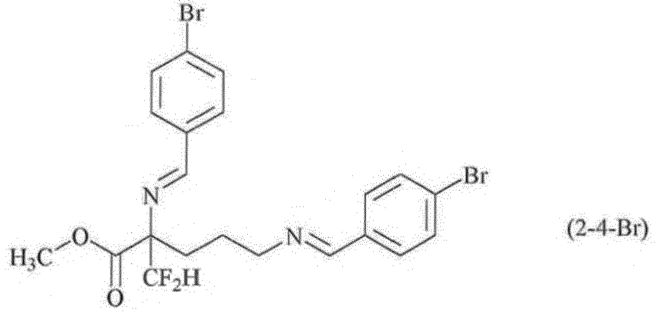

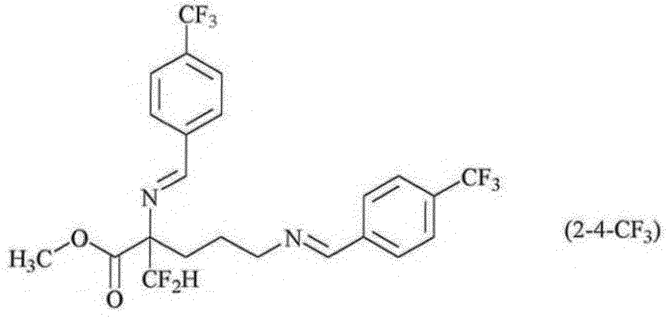

## Revendications

1. Procédé de préparation du composé de formule (II) 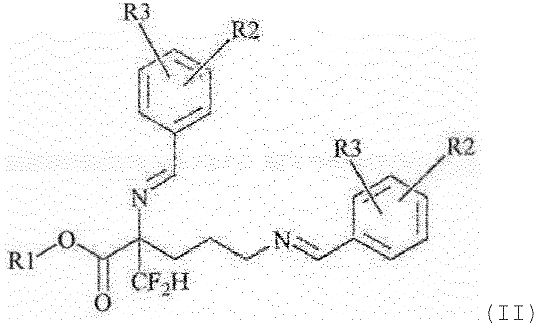 comprenant une réaction REAC1, dans laquelle le composé de formule (I) réagit avec CHF₃ en présence de bis(triméthylsilyl)amide de lithium ; 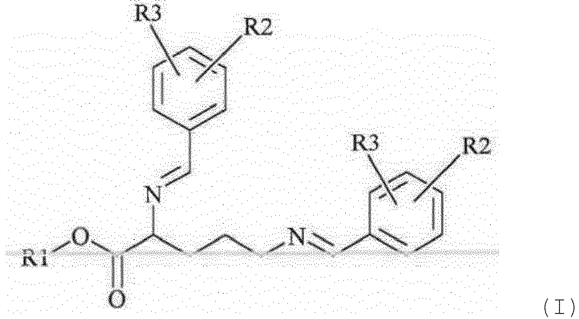
R1 est alkyle en C₁₋₄ ou benzyle ;
R2 et R3 sont identiques ou différents et sont choisis dans le groupe constitué de H, alcoxy en C₁₋₄, F, Cl, Br, I, NO₂, CN, CF₃, OH et alkyle en C₁₋₄.

2. Procédé selon la revendication 1, dans lequel R1 est méthyle, éthyle, isopropyle, n-butyle ou benzyle.

3. Procédé selon la revendication 1 ou 2, dans lequel R2 et R3 sont choisis dans le groupe constitué de H, alcoxy en C₁₋₄, F, Cl, Br, CN, CF₃ et alkyle en C₁₋₄.

4. Procédé selon une ou plusieurs des revendications 1 à 3, dans lequel
REAC1 est effectuée dans un solvant SOLV1, SOLV1 est un solvant qui est compatible avec le bis(triméthylsilyl)amide de lithium.

5. Procédé selon une ou plusieurs des revendications 1 à 4, dans lequel REAC1 est effectuée en continu.

6. Procédé selon une ou plusieurs des revendications 1 à 5, dans lequel le composé de formule (I) est préparé par une réaction REAC0, dans laquelle un composé de formule (V) réagit avec un composé de formule (IV) ; avec R1, R2 et R3 tels que définis dans la revendication 1.

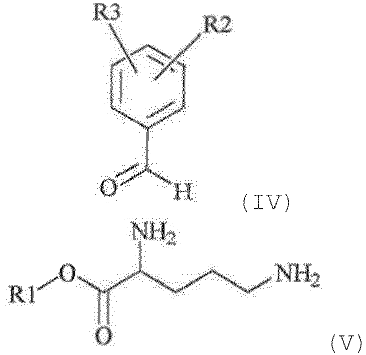

7. Procédé selon la revendication 6, dans lequel REAC0 est effectuée en présence d'une base BASE0.

8. Procédé selon la revendication 7, dans lequel BASE0 est une (alkyle en C₁₋₄)₃ amine.

9. Procédé selon une ou plusieurs des revendications 6 à 8, dans lequel
REAC0 est effectuée dans un solvant SOLV0.

10. Procédé selon la revendication 9, dans lequel SOLV0 est le chloroforme ou le dichlorométhane.

11. Procédé de préparation du composé de formule (3) 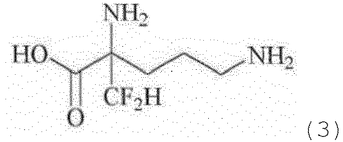 comprenant REAC1 et une déprotection du composé de formule (II) ;
REAC1 et le composé de formule (II) étant tels que définis dans la revendication 1.

12. Procédé selon la revendication 11, dans lequel la déprotection est effectuée avec un acide et en présence d'eau.

13. Procédé selon la revendication 12, dans lequel l'acide utilisé pour la déprotection est ACID2, ACID2 est HCl, H₂SO₄ ou l'acide acétique.

14. Procédé selon une ou plusieurs des revendications 11 à 13, dans lequel
la déprotection est effectuée en continu.

15. Procédé selon une ou plusieurs des revendications 11 à 14, dans lequel
REAC1 et la déprotection sont toutes deux effectuées en continu et consécutivement.

16. Composé de formule (2-4-Cl), composé de formule (2-4-Br) et composé de formule (2-4-CF₃).
